(19) **Europäisches Patentamt / European Patent Office / Office européen des brevets**

(11) **EP 2 246 702 B1**

(12) **EUROPEAN PATENT SPECIFICATION**

(45) Date of publication and mention of the grant of the patent:
**07.10.2015 Bulletin 2015/41**

(21) Application number: **08857449.6**

(22) Date of filing: **01.12.2008**

(51) Int Cl.:
**G01N 33/543** (2006.01)    **G01N 5/02** (2006.01)
**G01N 21/27** (2006.01)    **G01N 33/553** (2006.01)

(86) International application number:
**PCT/JP2008/071780**

(87) International publication number:
**WO 2009/072457 (11.06.2009 Gazette 2009/24)**

(54) **BIOSENSING METHOD USING COATED MAGNETIC MICROPARTICLES AND BIOSENSING DEVICE TO BE USED IN THE METHOD**

BIOSENSING-VERFAHREN UNTER VERWENDUNG BESCHICHTETER MAGNETISCHER MIKROPARTIKEL UND BEI DEM VERFAHREN ZU VERWENDENDE BIOSENSING-VORRICHTUNG

PROCÉDÉ DE BIODÉTECTION UTILISANT DES MICROPARTICULES MAGNÉTIQUES REVÊTUES ET DISPOSITIF DE BIODÉTECTION DEVANT ÊTRE UTILISÉ DANS LE PROCÉDÉ

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **03.12.2007 JP 2007311860**

(43) Date of publication of application:
**03.11.2010 Bulletin 2010/44**

(73) Proprietor: **Tamagawa Seiki Co., Ltd.**
**Nagano Pref. 395-8515 (JP)**

(72) Inventors:
• **HANDA, Hiroshi**
**Yokohama-shi**
**Kanagawa 226-0026 (JP)**
• **ABE, Masanori**
**Tokyo 152-0033 (JP)**
• **HATAKEYAMA, Mamoru**
**Yokohama-shi**
**Kanagawa 226-0026 (JP)**
• **SAKAMOTO, Satoshi**
**Yokohama-shi**
**Kanagawa 226-0026 (JP)**
• **NISHIO, Kosuke**
**Yokohama-shi**
**Kanagawa 226-0026 (JP)**
• **MOCHIZUKI, Yusuke**
**Yokohama-shi**
**Kanagawa 226-0026 (JP)**

(74) Representative: **Manitz, Finsterwald & Partner GbR**
**Martin-Greif-Strasse 1**
**80336 München (DE)**

(56) References cited:
**WO-A1-2006/079016    WO-A2-03/054523**
**JP-A- 5 203 651    JP-T- 2001 510 565**
**US-A1- 2006 257 958**

EP 2 246 702 B1

## Description

[TECHNICAL FIELD]

The present invention relates to a method for biosensing and more particularly relates to a method of biosensing using coated magnetic fine particles.

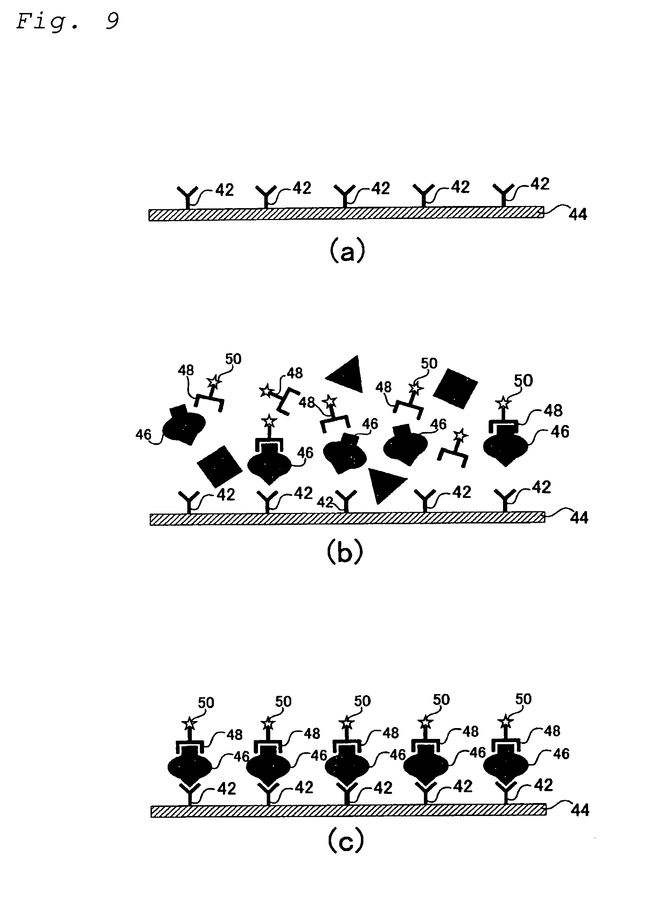

Conventionally in biosensing fields, a DNA chip using complementary bindings between polynucleotides having complementary sequences each other or a antibody chip using specific bindings occurring between antigens and antibodies etc. have been variously studied. In this regard, Japan Patent (Laid-Open) No. JP2005-533236 (Patent Literature 1) discloses methods for detecting and measuring antigens using a sandwiched ELISA. Fig. 9 shows a time sequence of the sandwiched ELISA disclosed in Patent Literature 1. As shown in Fig. 9(a), a first antibody 42 to which the antigen of the object of the detection is adhered to the substrate 44 to set the solid phase. Next as shown in Fig. 9(b), the substrate 44 is immersed into a sample solution including the antigen 46 followed by addition of a second antibody 48 which identify an other epitope different from the first antibody 42, In this point, the second antibody is marked by the molecular marker 50 such as enzymes or fluorescent materials. After passing sufficient reaction time, the substrate 44 is rinsed to remove non-reacted materials and the complex of substrate 44-first antibody 42- antigen 46-second antibody 48 on a surface of the substrate. Here, the molecular marker 50 bound to the second antibody is measured by a corresponding measuring system to detect indirectly the antigen 46.

As described above with the sandwich ELISA as an example, most of biosensing generally uses a affinity reaction between biomolecules such as an antigen-antibody reaction and many of such affinity reactions almost requires the reaction time not less than several hours such that this reaction step lowers a throughput of the sensing operation.

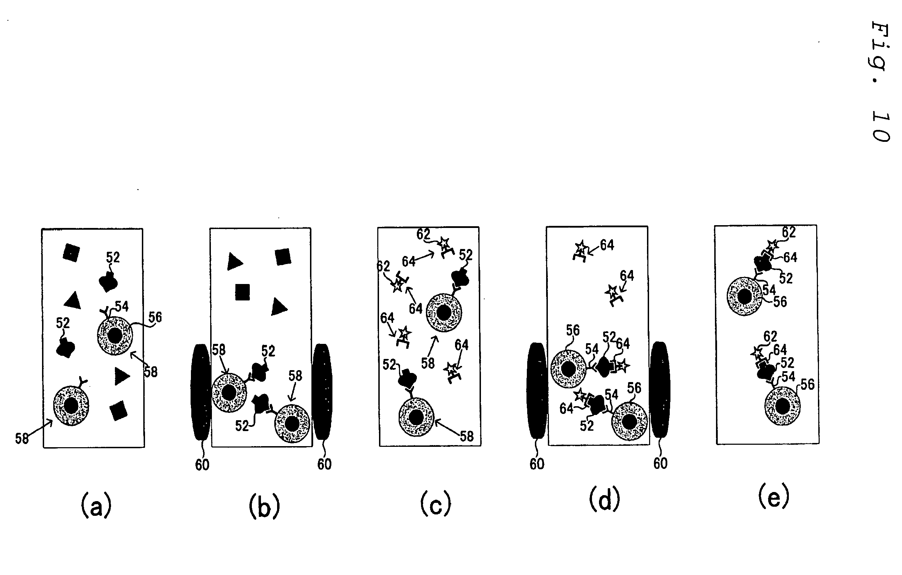

Furthermore, Japan Patent (Laid-Open) NO. JP2003-130880 (Patent Literature 2) discloses a different method of the sandwiched ELISA using magnetic particles. Fig. 10 shows a time sequence of the sandwiched ELISA using the magnetic particles disclosed in Patent Literature 2. As shown in Fig. 10 (a), in Patent Literature 2, the antibody-magnetic particle complex 58 formed by adsorbing the first antibody 54 which specifically binds to the antigen 52 to be an object of the detection to the magnetic particles 56 is added to the sample solution including the antigen 52 to be an abject of the detection and then is kept still for sufficient time to react the first antibody 54 and the antigen 52. Subsequently, as shown in Fig. 10 (b), the antibody-antigen complexes 58 are magnetically collected by the action of the magnet 60 and then are rinsed to remove unreacted substances. Further next as shown in Fig. 10 (c), the second antibody 64 marked with the molecular marker 62 is added to the sample solution only including the complex which formed by the antibody-magnetic particle complex 58 and the antigen 52 to react thereof sufficiently. As the results as shown in Fig. 10 (d),the complex of magnetic particle 56-first antibody 54-antigen 52-second antibody 64 is formed. Now, the magnetic particle 56 is magnetically collected again and is rinsed to remove the unreacted second antibody 64 and only the complex of magnetic particle 56-first antibody 54- antigen 52-second antibody 64 is rest as shown in Fig. 10 (e). Here by measuring the molecular marker 62 bound to the second antibody 64 using the corresponding measurement system to detect the antigen 52 indirectly.

However, Patent Literature 2 only omits a time-consuming centrifugal separation process by using the magnetic particles and then still requires the reaction time of several hours for the antibody antigen reaction in the processes shown in Figs. 10 (a) and (c) such that any solution for the problem above described with respect to Patent Literature 1 could not be provided at all. As described above, in the conventional biosensing technologies, the process step of the affinity reaction such as antibody-antigen reactions becomes a bottle neck and lowers the throughput and then it has been still requested a method for reducing the above reaction time.

On the other hand, recently magnetic particles have been took attention as a standard material used for biosensing. Though fluorescent substances are suspected to degrade the coloring thereof with respect to time elapse due to quenching of the fluorescence, the material property, i.e. magnetic property of the magnetic particles do not degrade with respect to the time elapse and an excellent measurement system by which the magnetism can be measured quickly and precisely has been established; furthermore, the latent usefulness is expected to the magnetic particles in relation to handling thereof using the magnetism such that further extensive applications of the magnetic particles is expected in the biosensing field.

With respect to the above backgrounds, Japan Patent (Laid-Open) No. JP2006-88131 (Patent Literature 3) discloses the novel polymer coated magnetic fine particles. The polymer coated magnetic fine particles have the average particle size from several tens nm to several hundreds nm and preferably have both of high dispersibility and magnetic responsibility which have been considered to be difficult in the polymer coated magnetic fine particles.

Patent Literature 1: Japan Patent (Laid-Open) No. 2005-533236

Patent Literature 2: Japan Patent (Laid-Open) No. 2003-130880

Patent Literature 3: Japan Patent (Laid-Open) No. 2006-88131

WO 03/054523 A2 discloses a method for determining the presence or for measuring an areal density of magnetic nanoparticles on a substrate comprising the steps of i) binding a target to selectively binding sites on the substrate, wherein the target sample is directly or indirectly labeled with magnetic nanoparticles, ii) sensing the presence of the bounds magnetic nanoparticles to a binding site to thereby determine the presence or density of the target labeled with magnetic nanoparticles, wherein the sensing step is carried out by extracting two signals derived from the magnetic field generated by nanoparticles bound to the one binding side using magneto-resistive sensor elements and determining the difference between the two signals

US 2006/0257958 A1 discloses a method of effecting a sandwich assay in a magnetically-assisted test strip apparatus comprising the steps of introducing a target analyte into a channel of the test strip apparatus, effecting a first reaction, during which the target analyte binds to a receptor bound to magnetic beads, passing a magnetic field along the length of the channel in order to influence the magnetic bead to move along the channel to a location viewable to a detection window, effecting a second reaction, during which the target analyte binds with a reagent, and analyzing the reaction product.

[SUMMARY OF INVENTION]

[TECHNICAL PROBLEM TO BE SOLVED BY INVENTION]

The present invention has been made by considering the above described problems in conventional techniques and an object of the present invention is to provide a novel biosensing method enabling the improvement of the throughput of the sensing while maintaining superior properties as a marker as a novel application development of the magnetic particles in the biosensing.

[MEANS FOR SOLVING TECHNICAL PROBLEM]

The inventors have been studied on the novel application development of the magnetic particles in the biosensing, and have been reached the idea that by immobilizing ligands or receptors to magnetic fine particles and the magnetic fine particles are forced to reaction fields of the affinity reaction by magnetic guiding in the affinity reaction of the biosensing so as to bring the affinity reaction, which is the velocity controlling factor in the sensing, to high rates. Further to the above, the present inventors have found that the usage of coated magnetic fine particles with having both of the high dispersibility and high magnetic responsibility as the above described magnetic fine particles makes the above affinity reaction quickly and high density so that relatively large signals may be obtained within significantly short time duration when compared to the conventional art.

Thus, according to the present invention, a biosensing method using an affinity reaction may be provided. The method comprises immobilizing a ligand of the affinity reaction to magnetic fine particles and magnetically and forcibly guiding the magnetic fine particles to a reaction field of the affinity reaction to bring the affinity reaction to high rates. The present method may further comprise the step of rinsing the reaction field under shaking. The present method may further comprise the step of rinsing by guiding the magnetic fine particles in a direction going far from the reaction field. In the present method, the magnetic fine particles may preferably have an average particle size from 3-400 nm and the magnetic fine particles are polymer coated magnetic fine particles. In the present invention, the magnetic fine particles may be the magnetic fine particles coated with molecules containing functional groups. In the present invention, the magnetic fine particles have a fluorescent function and the polymer coated fine magnetic particles are magnetic fine particles to which a fluorescent substance is introduced to a polymer layer by absorption. In the present invention, sensing may be conducted by using a magnetic sensor and the sensing may be conducted by using an optical detector, particularly by a fluorescence detector. Furthermore, according to the present invention, the sensing may be conducted by an SPR sensor using a surface plasmon resonance method or a mass detection sensor using a quartz resonation micro-balancing method. Further another aspect of the present invention, a biosensing apparatus using an affinity reaction is provided and the apparatus comprises i) a substrate on which a counter part substance of the affinity reaction is bound, and ii) a magnetic guiding means placed at the back of the substrate, the magnetic guiding means magnetically and forcibly guiding magnetic fine particles to which a ligand of the affinity reaction is immobilized thereon to a reaction field of the affinity reaction at the opposite side of the substrate, in which a fluorescent material is introduced to a polymer layer of the magnetic fine particles by absorption of the fluorescent material in the polymer layer.

Further according to the present invention, in a sandwiched ELISA method for detecting an antigen specifically binding to 2 antibodies, and the method may be **characterized in that** magnetic fine particles to which a second antibody is immobilized are magnetically and forcibly guiding to a substrate to which a first antibody is immobilized. Further according to the present invention, a method for detecting cancer cells may be provided and the method may comprise the steps of placing a tissue slice of a patient on the substrate and magnetically and forcibly guiding the magnetic fine particles to which the antibody binding specifically to a protein molecule being specifically expressed to cancer tissues.

[TECHNICAL ADVANTAGE OF INVENTION]

As described above, according to the present invention, the novel biosensing method may be provided; the method may have the superiority as the marker and the same time may improve the throughput of the biosensing as the novel application development of the magnetic particles in the biosensing. According to the biosensing method of the present invention, the relatively large signals may be obtained within significantly short time duration while attaining prompt and high precision analysis by using the magnetic particles as the marker because the affinity reaction may be preferably accelerated.

[BEST MODE FOR PRACTICING INVENTION]

Hereinafter the present invention will be described depending on embodiments depicted in drawings; however, the present invention must not be limited to the embodiments depicted in the drawings.

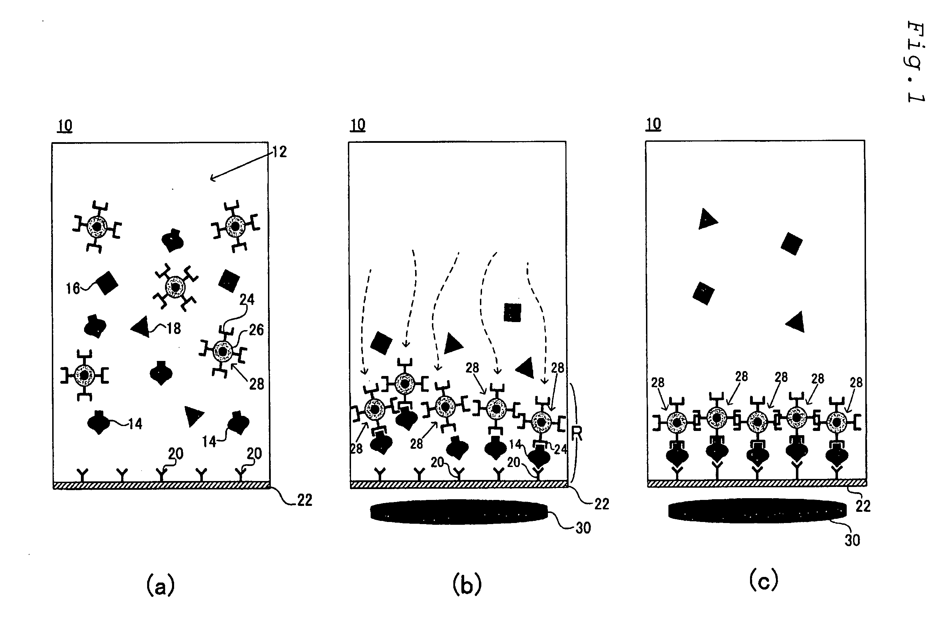

First of all, a mechanism of the biosensing method using the coated magnetic fine particles of the present invention is explained by using as the example the sandwich ELISA which is popularly used as one method of the biosensing. Fig. 1 shows the reaction system 10 of the sandwich ELISA in a time sequence. As shown in Fig. 1 (a), the sample solution 12 contains the antigen 14 as the object of detection and other protein 16 and protein 18 and the substrate 22 on which the first antibody 20 which binds specifically to the antigen 14 is adsorbed on the surface thereof to form a solid phase is immersed in the sample solution 12. In the conventional sandwich ELISA, a second antibody is contained which binds specifically to the antigen 14 to such reaction system and the second antibody has been marked by molecular markers such as enzymes or fluorescent substances. The present invention is characterized by the usage of antibody-magnetic fine particle complex 28 which is formed by immobilizing the second antibody 24 to the magnetic fine particles 26.

In the present invention, the magnetic fine particle 26 may be preferred that with excellent magnetic responsibility and also with the high dispersibility being near to mono-dispersion. According to the present invention, magnetic fine particles with the average particle size from 3nm to 400nm, more preferably with the average particle size from 10nm to 20nm are used. With respect to the magnetic fine particle with an extremely small size while having high magnetic responsibility and high dispersibility, it is considered that preparation of such magnetic fine particle could be difficult; however, the inventions disclosed in Japan Patent (Laid-Open) No.2006-88131, Japan Patent (Laid-Open) No.2006-313493, and Japan Patent (Laid-Open) No.2007-194233 all being assigned to the present applicant have provided such magnetic fine particle. Now, the magnetic fine particle 26 of the present invention will be detailed herein later.

Though the magnetic fine particle 26 used herein is markedly smaller than the conventional one, the diffusion coefficient thereof is $10^{-8}$ in the fine particle of which particle size is to be 200 nm; the value of diffusion coefficient is smaller by 2-orders than that of conventional molecular marker with the diffusion coefficient of $10^{-6}$ and hence, the reaction rate thereof becomes lower than that of the reaction system which uses the conventional molecular marker.

With regarding to this point, the inventors have adopted the construction in which the antibody-magnetic fine particle complex 28 is made approach to the reaction field by magnetically guiding the magnetic fine particles 26. This mechanism will be explained using Fig. 1(b).

Contradictory to the reaction system shown in Fig. 1(b), according to the present invention, the decrement of the reactivity due to the small diffusion coefficient may be supported by magnetic guiding of the antibody-magnetic fine particle complex 28 using the magnetic field generator 30. In the reaction system 10 shown in Fig. 1 (b), because the first antibody 20 is immobilized on the surface of the substrate 22 the neighborhood of the surface of the substrate 22 becomes the reaction field R. Therefore, the magnetic field generator 30 may be placed, in the present invention, for example, at the back of the substrate 22 to act the magnetic force to the reaction system 10, thereby guiding magnetically and forcibly the antibody-magnetic fine particle complex 28 to the neighborhood of the substrate 22, i.e. to the reaction field R. By adopting such construction, the sandwich reaction among antibody 20-antigen 14-antibody 24 may be accelerated and hence, as shown in Fig. 1(c), the antibody-magnetic fine particle complexes 28 may be adsorbed to the substrate 22 in high density. Here, the magnetic field generator 30 may be adequately selected from a permanent magnet or an electric magnet.

Furthermore, according to the present invention, because the average particle size of the magnetic fine particles 26 is markedly small from about 3nm to about 400nm, an effect of an impulse by flow of the reaction system 10 may be adequately avoided so that the stability of the antibody-magnetic fine particle complexes 28 after the adsorption may be kept. This means low possibility for loss of the specific bindings of the affinity reaction to ensure high sensing precision.

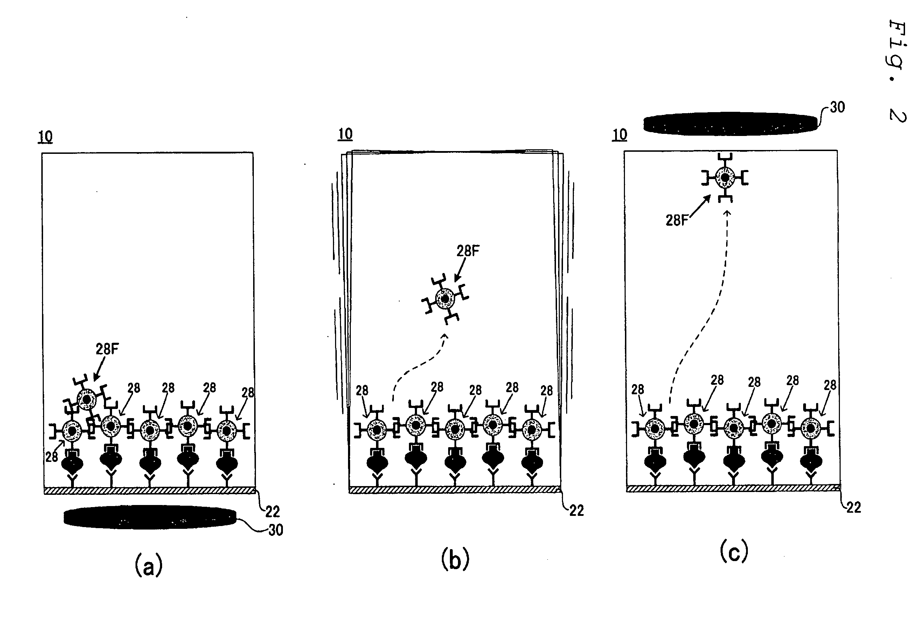

In addition, the biosensing method of the present invention also discloses based on the stability after the adsorption the method described hereunder in the point of view of enhancing sensing precision. Fig. 2 depicts a schematic illustration of the rinse process in the present biosensing method for removing selectively non-specific bindings being not derived from the affinity reaction by using the sandwiched ELISA reaction system 10 shown in Fig. 1. As indicated by the arrow in Fig. 2 (a), there are cases that the antibody-magnetic fine particle complexes 28F are adsorbed to the substrate 22 through the non-specific binding being not derived from the antigen-antibody reactions. The presence of such antibody-magnetic fine particle complexes 28F degrades the sensing precision and hence, such presence must be removed selectively. Here, according to the present invention as shown in Fig. 2(b), the rinse may be performed with shaking the reaction system 10 by using a plate shaker (not shown) etc. When rinsing, the magnetic field from the magnetic field generator 30 being placed at the back of the substrate 22 is preferably omitted. Then, the antibody-magnetic fine particle complexes 28F which were adsorbed onto the substrate 22 through the non-specific binding may be removed. On the other hand, the other antibody-magnetic fine particle complexes 28 adsorbed onto the substrate 22 through the specific binding based on the antigen-antibody reaction may not remove because the complex 28 does not largely influenced by the impulse of the flow of the reaction system 10 such that the above rinsing and recovering may remove selectively the non-specific bindings.

Furthermore, in the present invention, the rinsing may be performed with the magnetic guiding in the direction that the antibody-magnetic fine particle complexes 28F go far away from the reaction field R by placing the magnetic field generator 30 at the opposite side of the substrate 22 as shown in Fig. 2(c). In this case, the antibody-magnetic fine particle complexes 28F which are adsorbed on the substrate 22 through the non-specific binding may go off from the substrate 22 by the influence of the magnetic field such that the complexes may be collected magnetically at the side of the magnetic field generator 30. In turn, the specific binding force based on the antigen-antibody reaction may stronger than the influenced force of the magnetic field such that the above rinsing and recovering may remove selectively the non-specific bindings. In accordance with the present invention, to achieve higher sensing precision, it may be preferred that both the above shaking and the magnetic guiding are applied in the rinsing process.

As describe above, the biosensing method of the present invention has been described by using the reaction system of the sandwich ELISA as the example thereof; however, the present invention is not limited to the biosensing utilizing the antigen-antibody reaction and may be applicable to a wide range of biosensing methods utilizing the affinity reactions. The term "affinity reaction" herein includes the specific reactions between biomolecules (including the reaction with artificially modified biomolecule) such as, for example, the complementary bindings between nucleic acid DNA, the specific bindings between nucleic acid and proteins, the bindings between the proteins being relevant to signal communication system and the receptor proteins thereof, the specific bindings between proteins such as the bindings between "Protein A" or "Protein G" and the Fc regions of antibodies, the specific bindings between enzymes and the substrates thereof, the specific bindings between hormone molecules and the receptor thereof, the specific bindings between sugar chains and lectin etc. as well as the specific bindings between artificial antibody-like molecules such as aptamers etc. and biomolecules, the specific bindings between drugs or drug candidate materials and biomolecules, the specific bindings between avidin and biotin etc., the specific bindings between low molecular compounds and biomolecules (including the reaction with artificially modified biomolecules). The term "ligand" herein means any one of the elements composing the affinity reactions (specific bindings) and also means synthesized substances being capable of adsorbing through affinity forces to the other elements composing the reaction per se.

The application embodiments of the present invention may include such as, for example, the practical construction for accelerating the complementary bindings between nucleic acids in the so-called DNA chip to which the nucleic acid having a complementary base sequence to the nucleic acid to be an objective of the analysis is immobilized to the chip by immobilizing the nucleic acid of an objective of the analysis to the above magnetic fine particle to form forming a nucleic acid-magnetic fine particle complex and magnetically guiding the nucleic acid-magnetic fine particle complexes by acting the magnetic field from the back side of the chip when adding the nucleic acid-magnetic fine particle complexes to the chip.

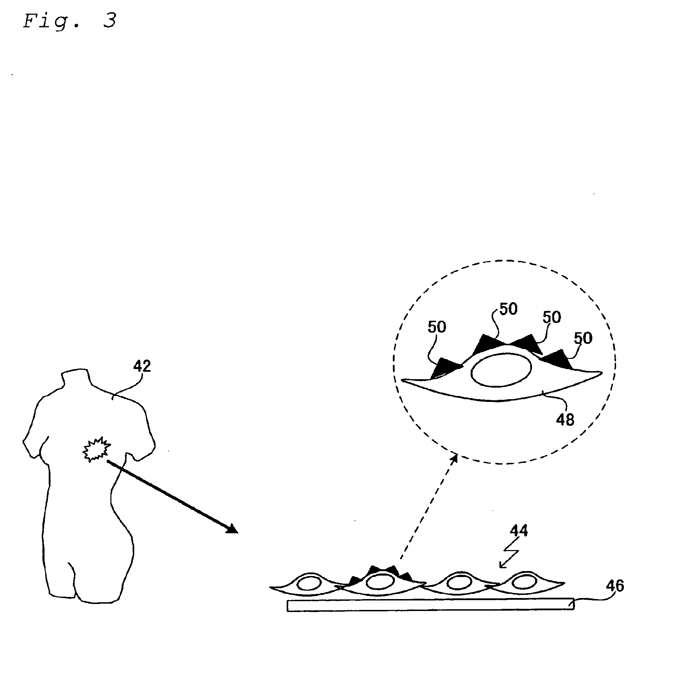

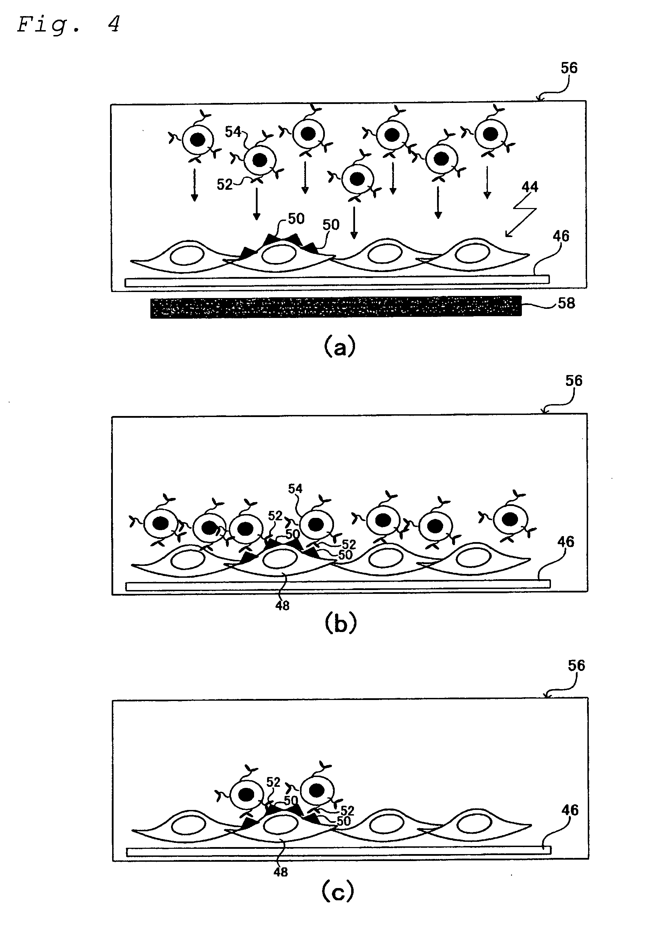

Furthermore, the present biosensing method may be directly applicable to samples rather than the cases of using the substrates to which the antibody or the nucleic acid are beforehand immobilized such as examples above described sandwich ELISA or DNA chip. Such application embodiments will be described using Figs. 3 and 4.

Generally, when metastasis of cancers is diagnosed in a medical diagnosis, a sentinel lymph node in the position being suspected to the metastasis of the cancer is extracted from the patient 42 as shown in Fig. 3 and the presence or not of protein molecules in the tissues which are specifically expressed in cancer tissues is examined. In the present embodiment, the tissue slice 44 extracted from the patient 42 is placed on the slide glass 46 to perform the biosensing utilizing the antigen-antibody reactions. If the cancer is metastasized, it is considered that the protein molecule 50 expressed specifically to the cancer tissue from the cross section of the cancer cell 48 is exposed.

Fig. 4 shows the sequence for diagnosing the presence or absence of the cancer cells about the tissue slice 44 extracted from the patient 42 using the biosensing method of the present invention. As shown in Fig. 4A(a), first the slide glass 46 onto which the tissue slice 44 is placed is set to the reaction vessel 56 within which the reactive solution including the magnetic fine particles 54 onto which the antibody 52 to the protein 50 is immobilized. Next, the magnetic field generator 58 is placed at the back of the slide glass 46 and the magnetic fine particles 54 are subjected to the magnetic guiding to attain prompt adsorption of the magnetic fine particles 54 on the surface of the cancer cell 48 through the antigen-antibody reaction between the protein molecule 50 and the antibody 52. Then, the rinsing is performed using the same sequence described about Figs. 1 and 2 to exclude the excess magnetic fine particles 54 as shown in Fig.

4(c). Lastly, the presence of the cancer cell is specified by the detection of the residual magnetic fine particles 54.

In the biosensing method using the present coated magnetic fine particles, the measurement system thereof may be a high sensitive magnetic sensor to realize highly reliable sensing by measuring the magnetism of the coated magnetic fine particles quickly and precisely. The magnetic sensor used herein may include such as for example, a hall element, a SQUID element, an MR element, a GMR element, a TMR element and an MI element. Furthermore, according to the present invention the SPR sensor which uses a surface plasmon resonance method or the mass detection sensor which uses a quartz resonator macro-balancing method may be adopted as the sensing system therefor.

Hereafter, the magnetic fine particle 26 used in the present biosensing method will be explained. Various applications of the coated magnetic fine particles to the biosensing field have been studied; however, the dispersibility so far and the responsibility to the magnetic field become trade-off and hence it has been considered that materials that satisfy the both properties. With respect to this point, the inventors of the invention disclosed in Japan Patent (Laid-Open) No. JP2006-88131 assigned to the present applicant has succeeded in preparing polymer coated magnetic fine particles which have significantly small particle sizes and which are coated excellently by polymers while having both of high dispersibility and high magnetic responsibility. In the present invention, the magnetic fine particles 26 may be selected from the polymer coated fine particles with the average particle size from 25 nm to 400 nm disclosed in JP2006-88131. Now, a preparation method of the above polymer coated magnetic fine particles will be reviewed. First, to hydrophilic ferromagnetic fine particles of the average particle size from 20 nm to 300 nm such as ferrite particles, hydrophobic materials such as fatty acids is adsorbed thereto to provide the hydrophobic property and then providing hydrophilic property by using a surface active agent having a nonionic hydrophilic group in order to suppress ionic strength. In turn, a monomer solution is emulsified by using a nonionic surface active agent and an ionic surface active agent. Glycidylmethacrylate and styrene may be used as monomers. Next, the above dispersion solution and the monomer emulsion are mixed to conduct an emulsion polymerization such that the uniform and stable polymer coating may be provided to preferably disperse ferromagnetic fine particles. Thus the polymer coated ferromagnetic fine particles with matched particle sizes and with excellent responsibility to the magnetic field may be prepared.

In order to immobilize ligands to the above described polymer coated ferromagnetic fine particles, the functional groups which are able to react with the ligands may be introduced to the above polymer coating. For example, epoxy group may be introduced to the polymer coating and the epoxy group may be treated by ammonia to introduce hydroxyl group and amino groups may be introduced. Furthermore, a monoethyleneglycoldiglycidylether(EGDE) molecule or a polyethyleneglycoldiglycidylether molecule may be bound to the above amino group to form spacers. By immobilizing biomolecules through such spacer three dimensional obstacles of the polymer coated ferromagnetic fine particles may be avoided.

In addition, the coated magnetic fine particles disclosed in Japan Patent Application No. 2007-194233 which is the former application of the present applicant and are coated by molecules including hydrophilic functional groups such as citric acid with the average particle size of 3 nm-40 nm may be used as the magnetic fine particle 26. Here, a preparation method of the above coated magnetic fine particles will be reviewed. First in a nonpolar solvent in which the magnetic fine particles coated with a fatty acid are dispersed, thiomalic acid etc. is added as a temporally coating substance to substitute the fatty acid coating with the temporal substance. Next, the magnetic fine particles coated with the temporal coating substance coating are dispersed in a polar solvent followed by adding molecules including hydrophilic functional groups such as citric acid and then the temporal coating substance is substituted with the above molecules including the functional groups. The aforementioned processes makes it possible to provide the coating with the molecules including the hydrophilic functional group to prepare the coated magnetic fine particles which are able to be dispersed preferably in polar solvents such as water. The magnetic particles have a matched particle size and have a uniform magnetic property such that an excellent quantitative performance may be obtained in magnetic sensing.

Here, the above described hydrophilic low molecular weight substances may include such as, for example, hydroxyl group containing polycarboxylic acid such as malic acid, citric acid and tartaric acid; amino group containing polycarboxylic acid such as aspartic acid and glutamic acid; phenolic hydroxyl group containing compounds such as catecol, salicylic acid and the derivatives thereof; macromolecules with relatively low molecular weight such as oligopeptides and proteins; thiol group containing compound such as cysteine; compounds including sulfonic acid group such as cysteic acid; compounds including phosphoric acid group; compounds including silanetriol. Furthermore, nucleic acids and the derivatives thereof, dextrane, polyvinylalcohol, polyacrylic acid, polyaspartic acid, polyglutamic acid, polylysine, alginic acid, hyaluronic acid, collagen, and gelatin and the derivatives thereof may be used.

Furthermore, the coated magnetic fine particles disclosed in Japan Patent (Laid-Open) No. 2006-313493 which is the former application of the present applicant and include a fluorescent substances in the coating polymer layer as the magnetic fine particle 26. A preparation method of the polymer coated magnetic fine particles including the fluorescent material will be reviewed as follows: First, the polymer coated magnetic fine particles are immersed in a nonpolar solvent in which the fluorescent material is dissolved to absorb the fluorescent substance in the polymer layer together with the solvent followed by removing the aqueous solvent from the polymer layer and then the polymer coated magnetic fine particles introduced therein the fluorescent material may be prepared. The above described fluorescent polymer coated magnetic fine particles may be detected and measured by the magnetic sensors and also used in the measurement using an optical detector such as fluorescence detector using the fluorescent function of the particles as the marker.

As described above, the present magnetic fine particles 26 have excellent magnetic responsibility and hence, it makes possible to accelerate the affinity reactions utilizing the magnetic guiding. In addition, the magnetic fine particles 26 have high dispersibility to be about monodispersity. Therefore, aggregation in the reaction field may be avoided such that it is expected difficult to cause sensing errors due to nonspecific bindings other than the affinity reaction.

The advantage of the present coated magnetic fine particle is to accelerate the affinity reaction which is the rate controlling factor in the biosensing by magnetically guiding the ligand immobilized magnetic fine particles to the neighborhood of the reaction field. By the present invention a rise time of the affinity reaction particularly is improved so that high signal within measuring ranges of the measurement system may be obtained in short time duration; as the result the present invention makes it possible to predict amounts of desired biomolecules in samples within a few minutes. This should suggest wide applicable ranges of the present invention; the present invention may have an application development in the medical field requiring urgent response. For example, the final excision range of the focus in cancer operations has been conventionally determined by eye views depending on doctor's experiences; however, the present invention may make it possible to obtain molecular level information about the range being suspected to the metastasis of the cancer (presence or not of the cancer specifically expressed protein molecules) in real time during the operation and to examine in situ the presence of the metastasis such that more precise determination of the excision may be possible.

[EXAMPLES]

Hereafter the present biosensing method utilizing the coated magnetic fine particles will be described more concretely using examples; however, the present invention must not limited to the examples described hereinafter.

(Example 1)

PREPARATION OF REACTION MODEL SYSTEM

The present example adopted a sandwich ELISA for detecting Brain Natriuretic Peptide; BNP as the reaction model. The reaction model was prepared by first adsorbing an antibody BC203 (From Shionogi & Co., Ltd.) for Brain Natriuretic Peptide (hereafter referred to BNP) on a gold substrate (5 mm x 5 mm) to form a solid phase while another antibody KY 2 (from Shionogi & Co., Ltd.) for BNP was immobilized on the surfaces of three kinds of magnetic fine particles; those were the polymer coated magnetic fine particle of the average particle size with about 200nm (hereafter referred to FG beads); citric acid coated magnetic fine particles with the average particle size of about 27nm; and commercially available DYNA beads (particle size of 2.8 micrometers, DYNA L, carboxylic group). Each of the antibody-magnetic fine particle complexes was prepared such that the amounts of antibody immobilized per a unit weight of the magnetic fine particle were same.

The immobilizing of the antibody onto the gold substrate was performed by providing antibody binding ability with reacting Z-tag protein including thiol groups at the terminal ends thereof to a self-organization molecular mono-layer film via a cross linker. The gold substrate was immersed in 20 microlitters of 1 mM PEG3-OH alkanethiol (Sensopath Technologies) ethanol solution for 24 hours at 37 Celsius degrees. Then the gold substrate was subjected to the reaction in 150 microlitters of DMSO solution with 50 mM PMPI(Pierce) for 2 hours in an ambient temperature. After that Z-tag protein including thiol groups at the terminal ends thereof was reacted in 200 microlitters of immobilization solution 1 (10 mM Hepes (pH7.0), 50 mM KCl, 1.0 mM EDTA) for 24 hours at 4 Celsius degrees. Thereafter 10 microgram of BC203 was reacted in 200 microlitters of immobilization solution 2 (10 mM Hepes(pH7.0),50 mM KCl, 1.0 mM EDTA, 0.1% Tween20) for 4 hours at 4 Celsius degrees to immobilize thereof on the gold substrate. All of the reactions was conducted by immersing the gold substrate in an Eppendorf tube.

The immobilization of the antibody on the magnetic fine particle was conducted by coupling the carboxyl groups on each surfaces to the amino groups of the KY2 antibody. 1 mg of the FG beads, the citric acid coated magnetic fine particles of the average particle size of 27 nm, the DYNA beads(particle size of 2.8 micrometers, DYNAL) were each gathered and dispersed in 1 ml of DMF which was adjusted to 0. 5M EDC (NAKARAI TESQUE, Inc.) and 0.5M NHS (PEPTIDE INSTITUTE INC.) to conduct the reaction for 2 hours at the ambient temperature. After that 10 microgram of KY2 antibody was reacted in the immobilization solution (10mMHepes (pH7.0), 50 mM KCl, 1.0 mM EDTA) for 2 hours at 4 Celsius degrees to prepare KY2-FG beads complexes, KY2-citric acid coated fine particle complexes, and KY2-DYNA beads complexes.

(2) EXAMINATION OF REACTION TIME

In wells of a 48-well plate, the gold substrates with adsorbed and immobilized BC203 were placed and each of 200 microlitters of the reaction solution (10 mM Hepes(pH7.9), 50 mM KCl, 1.0 mM EDTA, 0.1% Tween20, 0.03% skim-milk) was filled therein. Next, 1 microgram of BNP (PEPTIDE INSTITUTE, INC.) was added to each of the wells and was shaken for 5 minutes on a plate shaker for ELISA and the KY2-FG beads complexes were added on the gold substrate being immersed in the above reaction solution. Then, the 48 well plate was kept still for predetermined times (1 min., 10 min., and 100 min.) in the condition that a magnet was placed adjacent to the lower side thereof. After the predetermined time durations passed, the gold substrates were rinsed for 3 times by using the rinsing solution (10 mM Hepes(pH7.9),50 mM KCl, 1.0 mM EDTA, 0.1% Tween20). Lastly, each of the gold substrate was rinsed by Milli Q and was observed by a scanning electron microscope. In addition, experiments applying the similar procedures to the reaction system without adding BNP were conducted as backgrounds and furthermore, experiments applying the similar procedures to the reaction system without using the magnet were conducted as comparative example. The gold substrates after the reactions for the background and the comparative examples were observed by the scanning electron microscope. A surface coverage densities (%) were calculated from observation results of the scanning electron microscope. In the present example, the surface coverage density (%) was defined by the following formula (1) and 5 views observed in the scanning electron microscope were averaged:

[Formula 1]

$$\text{surface coverage density (\%)} = \frac{[\pi \times (\text{particle radius})^2] \times \text{particle numbers}}{\text{area per view}} \times 100 \quad (1)$$

Table 1 listed hereunder shows the relation between the surface coverage density (%) of the FG beads and the reaction times (1 min., 10 min., 100 min.,).

[Table 1]

|  | presence of magnet | | | absence of magnet | | |
|---|---|---|---|---|---|---|
|  | 1min | 10min | 100min | 1min | 10min | 100min |
| with BNP (%) | 15.07 | 20.64 | 50.65 | 0.43 | 2.04 | 3. 95 |
| without BNP (%) | 1.19 | 2.40 | 6.64 | 0.03 | 0.04 | 0.18 |

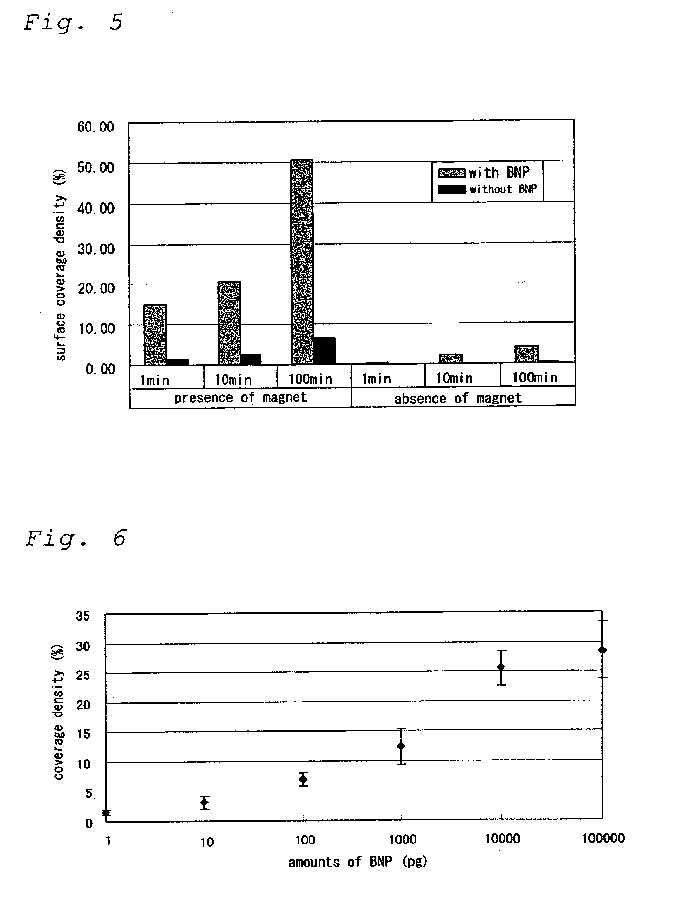

In Table 1, the results of the examples (presence of the magnet) are listed in the left-hand columns; the results of the comparative examples (absence of the magnet) are listed in the right-hand columns and the background values are listed in the lower row. The results of Table 1 were summarized in Fig. 5. As shown in Table 1 and Fig. 5, the surface coverage densities (%) of the magnetic guiding reaction system (presence of the magnet) had significantly higher values than those of the non-magnetic guiding system (absence of the magnet) in the same reaction time durations. For example, when the results after the reaction time passed for 1 min. were considered, the surface coverage density (%) of the reaction system with the magnetic guiding (presence of the magnet) has the high value as high as 30 times of that in the reaction system without the magnetic guiding (absence of the magnet). Furthermore, in the reaction system with magnetic guiding (presence of the magnet) the reaction time of only 1 min. showed a meaningful difference from the background value such that is is has been confirmed that the present invention could obtain reliable data within a few minutes.

(3) EXAMINATION OF MEASUREMENT PRECISION

Experiments were conducted with the addition of different amounts of BNP (1-105 picograms) using the same conditions applied to the above reaction systems and the surface coverage densities (5) were calculated for each reaction systems. Fig. 6 shows the relation between the BNP amounts (picogram) added to the reaction systems and the surface coverage density (%). As shown in Fig. 6, as the BNP amounts (picogram) are increased, the surface coverage densities (%) are increased and a certain concentration dependency was confirmed.

(4) EXAMINATION OF ADSORPTION STABILITY

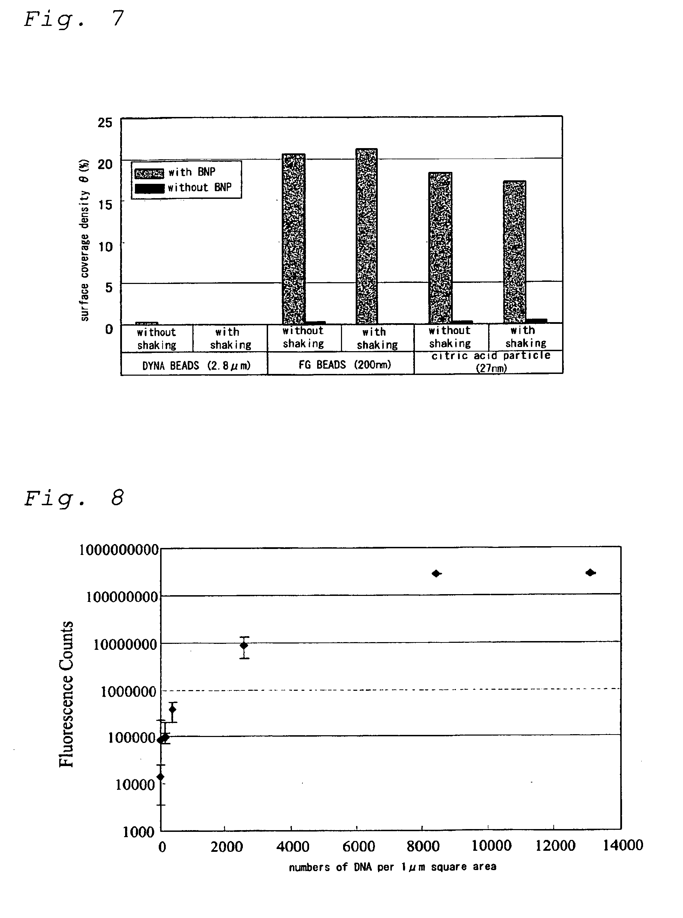

With respect to 3 kinds of the magnetic fine particles having different particle sizes, the adsorption stabilities to the substrate were examined and compared. To the well in the same condition described above, each of KY2-citric acid coated fine particle complexes (particle size of 27 nm), KY2-FG beads complexes (particle size of about 200 nm), and KY2-DYNA beads complexes (particle size of 2.8 micrometers) was added and the reaction was conducted for about 10 minutes in the condition that the magnetic force was applied along with the direction perpendicular to the gold substrate surface. Then the surface coverage densities (%) were calculated for each of the reaction systems. In addition, similar 3 reaction systems separately prepared were shaken on the plate shaker for ELISA (NISSIN) for 10 min. to conduct the reaction and then the surface coverage densities (%) were calculated for each of the reaction systems. Table 2 listed hereunder shows the surface coverage densities (%) of the above described 3 complexes and the results of Table 2 are shown in Fig. 7.

[Table 2]

| | DYNA BEADS (2.8 $\mu$m) | | FG BEADS (200nm) | | citric acid particles (27nm) | |
|---|---|---|---|---|---|---|
| | without shaking | with shaking | without shaking | with shaking | without shaking | with shaking |
| with BNP (%) | 0.33 | 0.073 | 20.6 | 21.1 | 18.2 | 17.2 |
| without BNP (%) | 0.0043 | 0.0026 | 0.31 | 0.035 | 0.35 | 0.51 |

As shown in Table 2 and Fig. 7 the reaction system using the KY2-DYNA beads complexes (particle size of 2.8 micrometers) showed lower surface coverage densities (%) in spite of applying the magnetic guiding. In turn, the reaction systems using KY2-citric acid coated fine particle complexes (particle size of 27 nm) and KY2-FG beads complexes showed the high surface coverage densities (%) as high as 200 times than that of the KY2-DYNA beads complexes (particle size of 2.8 micrometers).

In addition, when the reaction systems with or without the shaking during the reaction were compared. With respect to the reaction systems using DYNA beads complexes (particle size of 28 nm), the case with the shaking, the surface coverage density (%) was about one-fifth of that for the reaction system without the shaking. Contradictory to the above, the reaction systems using KY2-citric acid coated fine particle complexes (particle size of 27 nm) and KY2-FG beads complexes (particle size of 200 nm) did not show meaningful differences in the surface coverage densities (%). From the above results, the present biosensing method may achieve the high density adsorption of the fine particle by using the magnetic particle having the particle size of micrometers order and may hardly provide the sensing errors because the bindings once formed between the antigen and the antibody could not dissociate by the influence of the impulse associated to the rinsing process.

(EXAMPLE 2)

IN the present example, DNA hybridization was adopted as the reaction system. Single chain DNA (from TSUKUBA ORIGO SERVICE Co. , LTD.) of 35 bases was immobilized on fluorescent FG beads having the particle size of 200 nm to prepare a DNA-fluorescent FG beads complexes. The complementary chain DNA (35 bases) for the single chain DNA which was immobilized on the FG beads was adsorbed on a glass substrate (5 mm x 5 mm) to form the solid phase. In the wells of a 48 holes plate, the glass substrates with the complementary chain DNA in the solid phase were placed and 200 microlitters of the reaction solution (10 mM Hepes(pH7.9), 50 mM KCl, 1.0 mM EDTA, 0.1%, Tween20) was filled therein. The DNA-fluorescent FG beads complexes with different concentration (DNA molecules/1 micrometers square) were added to the glass substrates immersed in the reaction solution and were shaken for 1 min. by the plate shaker for ELISA (NISSIN) while placing the magnet at the neighborhood at the lower side of the 48 well plate. After rinsing of 3 times with the rinsing solution) 10 mM Hepes(pH7.9), 50 mM KCl, 1.0 mM EDTA, 0.1% Tween20), the DNA-fluorescent FG beads complexes were observed by a fluorescence microscope and fluorescence count values were measured. Fig. 8 shows the relation between densities (DNA molecules/1 micrometer square) of the DNA-fluorescent FG beads complexes added to the reaction system and the fluorescence count value. As shown in Fig. 8, the fluorescence count values increase as the densities of the DNA-fluorescent FG beads increase and then, a certain concentration dependency was confirmed.

As described above, the present invention may provide a novel biosensing method as the novel application development of the magnetic particles in the biosensing by positively using the superiority as the marker thereof while improving the throughputs of the biosensing. The biosensing method of the present invention may be expected to provide significant contributions for improvement of work efficiency in the biochemical research fields and clinical diagnosis fields.

[BRIEF DESCRIPTION OF DRAWINGS]

[Fig. 1] Fig. 1 shows a time sequence of a reaction system of a sandwich ELISA to which the present invention is applied.

[Fig. 2] Fig. 2 shows a rinsing process of the present biosensing method.

[Fig. 3] Fig. 3 shows a schematic view of a tissue slice extracted from a patient.

[Fig. 4] Fig. 4 shows a process sequence for examining presence or not of cancer cells about a tissue slice of a patient using the present biosensing method.

[Fig. 5] Fig. 5 shows a relation between a surface coverage density (%) of FG beads and reaction time.

[Fig. 6] Fig. 6 shows a relation between BNP amounts (pg) added to a reaction system and a surface coverage density (%).

[Fig. 7] Fig. 7 shows a relation between a particle size of magnetic particles and a surface coverage density (%).

[Fig. 8] Fig. 8 shows a relation between densities of DNA-fluorescent FG beads complexes added to a reaction system and fluorescence count values.

[Fig. 9] Fig. 9 shows a time sequence of a conventional sandwich ELISA process.

[Fig. 10] Fig. 10 shows a time sequence of a conventional sandwich ELISA process using magnetic fine particles.

[DESCRIPTION OF SIGNS]

10-reaction system of sandwich ELISA, 12-sample solution, 14-antigen, 16-protein, 18-protein, 20-first antibody, 22-substrate, 24-second antibody, 26-magnetic fine particle, 28-antibody-magnetic fine particle complex, 30-magnetic field generator, 42-patient, 44-tissue slice, 46-slide glass, 48-cancer cell, 50-protein molecule, 52-antibody, 54-magnetic fine particle, 56-reaction vessel, 58-magnetic field generator

**Claims**

1. A biosensing method using an affinity reaction comprising the steps of:

   immobilizing a ligand of the affinity reaction to magnetic fine particles,
   and magnetically and forcibly guiding the magnetic fine particles to a reaction field of the affinity reaction to bring the affinity reaction to high rates,
   **characterized in that** the magnetic fine particles are polymer coated magnetic fine particles, in which a fluorescent material is introduced to a polymer layer of the magnetic fine particles by absorption of the fluorescent material in the polymer layer.

2. The method of claim 1, wherein the method further comprising the step of rinsing the reaction field under shaking.

3. The method of claim 1 or 2, wherein the method further comprising the step of rinsing by guiding the magnetic fine particles in a direction going far from the reaction field.

4. The method of any one of claims 1-3, wherein the magnetic fine particles have an average particle size from 3-400 nm.

5. The method of any one of claims 1-4, wherein the magnetic fine particles are the magnetic fine particles coated with molecules containing functional groups.

6. The method of claim 1 or 5, wherein the magnetic fine particles have a fluorescent function.

7. The method of any one of claims 1-6, wherein sensing is conducted by using a magnetic sensor.

8. The method of claim 5 or 6, wherein sensing is conducted by using an optical detector.

9. The method of claim 8, wherein the optical detector is a fluorescence detector.

10. The method of any one of claims 1-5, wherein the sensing is conducted by an SPR sensor using a surface plasmon resonance method or a mass detection sensor using a quartz resonation micro-balancing method.

11. A biosensing apparatus using an affinity reaction comprising:

    a substrate on which a counter part substance of the affinity reaction is bound; and
    a magnetic guiding means placed at the back of the substrate, the magnetic guiding means magnetically and forcibly guiding magnetic fine particles to which a ligand of the affinity reaction is immobilized thereon to a reaction field of the affinity reaction at the opposite side of the substrate,
**characterized in that** the magnetic fine particles are polymer coated magnetic fine particles, in which a fluorescent material is introduced to a polymer layer of the magnetic fine particles by absorption of the fluorescent material in the polymer layer.

**Patentansprüche**

1. Biosensingverfahren unter Verwendung einer Affinitätsreaktion, welches die nachfolgenden Schritte umfasst:

   Immobilisieren eines Liganden der Affinitätsreaktion an feine magnetische Partikel
   und magnetisches sowie gezwungenes Führen der feinen magnetischen Partikel zu einem Reaktionsfeld der Affinitätsreaktion, um die Affinitätsreaktion auf hohe Raten zu bringen,
   **dadurch gekennzeichnet, dass** die feinen magnetischen Partikel mit Polymer beschichtete feine magnetische Partikel sind, in welche ein Fluoreszenzmaterial an eine Polymerschicht der feinen magnetische Partikel durch Absorption des Fluoreszenzmaterials in der Polymerschicht eingeführt sind.

2. Verfahren nach Anspruch 1, wobei das Verfahren des Weiteren den Schritt des Spülens des Reaktionsfeldes unter Schütteln umfasst.

3. Verfahren nach Anspruch 1 oder 2, wobei das Verfahren des Weiteren den Schritt des Spülens durch Führen der feinen magnetischen Partikel in eine Richtung, welche über das Reaktionsfeld hinausgeht, umfasst.

4. Verfahren nach einem der Ansprüche 1 bis 3, wobei die feinen magnetischen Partikel eine durchschnittliche Partikelgröße zwischen 3 und 400 nm aufweisen.

5. Verfahren nach einem der Ansprüche 1 bis 4, wobei die feinen magnetischen Partikel die feinen magnetischen Partikel sind, welche mit Molekülen, welche funktionelle Gruppen umfassen, beschichtet sind.

6. Verfahren nach Anspruch 1 oder 5, wobei die feinen magnetischen Partikel eine Fluoreszenzfunktion aufweisen.

7. Verfahren nach einem der Ansprüche 1 bis 6, wobei das Sensing durch Verwendung eines Magnetsensors durchgeführt wird.

8. Verfahren nach Anspruch 5 oder 6, wobei das Sensing unter Verwendung eines optischen Detektors durchgeführt wird.

9. Verfahren nach Anspruch 8, wobei der optische Detektor ein Fluoreszenzdetektor ist.

10. Verfahren nach einem der Ansprüche 1 bis 5, wobei das Sensing durch einen SPR-Sensor unter Verwendung eines Oberflächenplasmonresonanzverfahrens oder eines Massendetektionssensors unter Verwendung eines mikrobalancierenden Quarzresonatorverfahrens durchgeführt wird.

11. Biosensingvorrichtung unter Verwendung einer Affinitätsreaktion umfassend:

    ein Substrat, auf welches eine Gegenstücksubstanz der Affinitätsreaktion gebunden ist und
    ein Mittel zum magnetischen Führen, welches an der Rückseite des Substrats platziert ist, wobei das Mittel zum magnetischen Führen feine magnetische Partikel, an die ein Ligand der Affinitätsreaktion immobilisiert ist, magnetisch und gezwungen zu einem Reaktionsfeld der Affinitätsreaktion an der gegenüberliegenden Seite des Substrats führt,
    **dadurch gekennzeichnet, dass** die feine magnetischen Partikel mit Polymer beschichtete feine magnetische Partikel sind, in denen ein Fluoreszenzmaterial auf eine Polymerschicht der feinen magnetischen Partikel durch Absorption des Fluoreszenzmaterials in der Polymerschicht eingeführt sind.

**Revendications**

1. Méthode de biodétection utilisant une réaction d'affinité comprenant les étapes consistant à :

   immobiliser un ligand de la réaction d'affinité sur des particules fines magnétiques,
   et guider de manière magnétique et avec force les particules fines magnétiques vers un champ de réaction de la réaction d'affinité pour amener la réaction d'affinité à des taux élevés,
   **caractérisé en ce que** les particules fines magnétiques sont des particules fines magnétiques enduites de polymère, dans lesquelles un matériau fluorescent est introduit sur une couche de polymère des particules fines magnétiques par absorption du matériau fluorescent dans la couche de polymère.

2. Méthode selon la revendication 1, la méthode comprenant en outre l'étape de rinçage du champ de réaction sous agitation.

3. Méthode selon la revendication 1 ou 2, la méthode comprenant en outre l'étape de rinçage par guidage des particules fines magnétiques dans un sens s'éloignant du champ de réaction.

4. Méthode selon l'une quelconque des revendications 1 à 3, dans laquelle les particules fines magnétiques ont une taille de particule moyenne de 3 à 400 nm.

5. Méthode selon l'une quelconque des revendications 1 à 4, dans laquelle les particules fines magnétiques sont des particules fines magnétiques enduites avec des molécules contenant des groupes fonctionnels.

6. Méthode selon la revendication 1 ou 5, dans laquelle les particules fines magnétiques ont une fonction fluorescente.

7. Méthode selon l'une quelconque des revendications 1 à 6, dans laquelle la détection est réalisée en utilisant un capteur magnétique.

8. Méthode selon la revendication 5 ou 6, dans laquelle la détection est réalisée en utilisant un détecteur optique.

9. Méthode selon la revendication 8, dans laquelle le détecteur optique est un détecteur de fluorescence.

10. Méthode selon l'une quelconque des revendications 1 à 5, dans laquelle la détection est réalisée par un capteur SPR en utilisant une méthode de résonance plasmonique de surface ou un capteur de détection de masse en utilisant une méthode de micro-équilibrage par résonance de quartz.

11. Appareil de biodétection utilisant une réaction d'affinité comprenant :

    un substrat sur lequel une substance de contre-partie de la réaction d'affinité est liée ; et
    un moyen de guidage magnétique placé sur l'arrière du substrat, le moyen de guidage magnétique guidant de manière magnétique et avec force les particules fines magnétiques sur lesquelles un ligand de la réaction d'affinité est immobilisé vers un champ de réaction de la réaction d'affinité sur le côté opposé du substrat,
    **caractérisé en ce que** les particules fines magnétiques sont des particules fines magnétiques enduites de polymère, dans lesquelles un matériau fluorescent est introduit sur une couche de polymère des particules fines magnétiques par absorption du matériau fluorescent dans la couche de polymère.

*Fig.1*

(a)

(b)

(c)

Fig. 2

(a)          (b)          (c)

EP 2 246 702 B1

Fig. 3

*Fig. 4*

(a)

(b)

(c)

*Fig. 5*

*Fig. 6*

*Fig. 7*

*Fig. 8*

Fig. 9

(a)

(b)

(c)

Fig. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005533236 A
- JP 2003130880 A
- JP 2006088131 A
- WO 03054523 A2
- US 20060257958 A1
- JP 2006313493 A
- JP 2007194233 A